# EUROPEAN PATENT SPECIFICATION

(11) **EP 0 391 896 B1**
(45) Date of publication and mention of the grant of the patent: **02.03.1994**
(21) Application number: 88904570.4
(22) Date of filing: 20.05.1988
(51) Int. Cl.: A61K 9/16, A61K 9/72

(54) **ENHANCED UPTAKE DRUG DELIVERY SYSTEM**
ABGABESYSTEM ZUR ERHÖHTEN ARZNEISTOFFAUFNAHME
SYSTEME D'ADMINISTRATION D'UN MEDICAMENT FACILITANT SA FIXATTION

(30) Priority: 22.05.1987 GB 8712176
(43) Date of publication of application: 17.10.1990
(73) Proprietor: DANBIOSYST U.K. LIMITED, University Boulevard Nottingham (GB)
(72) Inventor: ILLUM, Lisbeth 19 Cavendish Crescent North, Nottingham NG7 1BA (GB)
(74) Representative: Bassett, Richard Simon
(86) International application number: GB8800396
(87) International publication number: WO8809163

(56) References cited:
- EP-A- 122 036
- EP-A- 257 915
- FR-A- 2 081 353
- GB-A- 2 176 105
- JOURNAL OF PHYRMACEUTICAL SCIENCES, vol. 72, no. 9, September 1983, American Pharmaceutical Association; L.ILLUM et al., pp. 1086-1089#
- CHEMICAL ABSTRACTS, vol. 107, no. 20, 16 November 1987, Columbus, OH (US); L.ILLUM, p. 488, no. 183492g#
- CHEMICAL ABSTRACTS, vol. 107, no. 24, 14 December 1987, Columbus, OH (US); L.ILLUM et al., p. 359, no. 223162h#

## Description

### FIELD OF THE INVENTION

The present invention relates to drug delivery systems and more particularly to a system which enhances the uptake of active drug material, particularly high molecular weight materials, especially from the nasal cavity.

References will be made to technical papers and other disclosures within this field which are included for purposes of explanation.

European patent applications 023,359 and 122,036 describe a powdery pharmaceutical composition for application to the nasal mucosa and methods for administration thereof. The pharmaceutical composition allows polypeptides and derivatives thereof to he effectively absorbed through the nasal mucosa. Similarly US Patent 4250163 describes a method for administering a medicament to the nasal mucosa where the preferred composition has mucoadhesive properties. European Patent application 128,831 has described how the use of biocompatible water soluble amphiphilic steroids other than natural bile salts are capable of increasing drug permeability across body surfaces to include the nose. The German Patent 2620446 describes an aqueous insulin preparation for nasal application containing a penetration enhancer in the form of an amphoteric, anionic or nonionic surface active agents, saponin, bile salts or surfactin. European patent application 230,264 describes an aqueous nasal drug delivery system for vaccines containing a high molecular weight drug, a gelling agent (e.g. hydroxy-ethylcellulose) and in some cases other additives (e.g. surfactants, glycerol, polyethyleneglycol).

None of the above patents and applications describes the use of microspheres for nasal administration nor the combination of a microsphere and an enhancing agent or other adjuvants that would be expected to provide enhanced bioavailability.

A microsphere preparation for nasal delivery has been described in PCT/GB86/00726 however this refers to materials having ion exchange properties and for one specific drug sodium chromoglycate for local effect rather than delivery to the general circulation.

At the present time the nose is being proposed as an alternative route for the delivery of drugs that will act within the systemic circulation. Particular attention is being focused on products of biotechnology, namely the peptides and proteins. Other drugs that are being suggested are those that are poorly absorbed orally or are extensively metabolised either in the gastrointestinal tract itself or are subjected to first pass metabolism in the liver.

Nasal delivery is considered to have promise for the following reasons.
1. The nose has a large surface area available for drug absorption due to the coverage of the epithelial surface by numerous microvilli.
2. The subepithelial layer is highly vascularized.
3. The venous blood from the nose passes directly into the systemic circulation and therefore avoids the loss of drug by first pass metabolism in the liver.

A wide variety of drugs has now been tested for bioavailability after administration via the nasal route. Some drugs appear to be absorbed effectively and show bioavailabilities comparable to the intravenous route. However, most drugs show a low bioavailability when administered intranasally, but there are exceptions. The natural steroid progesterone is largely ineffective when administered orally. When given by the nasal route it is absorbed effectively with a bioavailability similar to that for an intravenous injection; the peak concentration appearing after approximately 6 minutes. If progesterone is given via the oral route then published data suggest that the bioavailability is of the order of 1.2% as compared to IV administration (1). The second example is the beta-blocker propranolol. This drug is metabolised extensively in the liver and possibly in the gut wall when administered orally. When the drug is given intranasally in a simple solution, plasma levels identical to intravenous administration can be obtained (2).

Insulin, a drug that has been studied extensively for intranasal delivery, can be delivered across the nasal membrane but the absorption efficiency is normally about 5% of the adminstered dose. Absorption can be improved by the use of so-called absorption enhancers. For example in a study by Salzman insulin was administered in the presence of a surfactant, Laureth 9 (3). Not only was a clear dose response relationship obtained but also the peak level appeared rapidly. The potency of the intranasal insulin was approximately 1/10th that of intravenous administered insulin. Clearly if insulin can be delivered to patients in a safe and reliable way by nasal administration then such systems could have potential for administration with meals in type 1 diabetes.

Chien and Chang (4) have summarised the absorptive capacity of the nasal routes for a variety of drug substances. It will be noted that those materials of high molecular weight, i.e. peptides and proteins are normally poorly absorbed via the nasal route. Also is noted the fact that most of the compounds, both those with high and low absorption efficiencies, show peak plasma levels within approximately 30 minutes. Thus absorption, whatever its extent, appears to be rapid but does not last for a particularly long time. This indicates that the drug may either be removed from the site of absorption or, if sufficiently labile, is degraded before further absorption can occur.

### Factors affecting systemic absorption of drugs from the nose

The rapid clearance of nasal sprays from the nose can probably be considered to be a major factor in influencing loss of drugs from potential absorption surfaces. In addition, for the case of peptides and proteins, enzymatic degradation of the drug and molecular size may also have a role in giving low bioavailabilities.

Most workers in the field of nasal delivery have attempted to overcome the problem of inefficient absorption of drugs by using absorption enhancers e.g. in the form of bile salts or surfactants to modify the properties of the nasal mucosa thereby enhancing uptake. A typical example is the investigation described by Hanson et al (5) on the nasal delivery of the peptide salmon calcitonin. Here she showed clearly that a significant increase in plasma calcitonin could occur when the drug was given in combination with a surfactant. Thus, without enhancer only trace amounts of calcitonin appeared in the plasma whereas with enhancer involved the AUC increased 10 fold. Similarly, the striking effect of increasing amounts of bile salt (sodium deoxycholate) on the absorption of insulin has been well described by Gordon and others (6).

### Controlled release systems for the nose

Illum et al (7) have chosen microspheres made from materials that are known to swell in contact with water to form a gel-like layer with good bioadhesive properties. Thus, due to their adherence to the nasal mucosa they could well modify clearance. The materials selected included albumin, starch and the ion exchange material DEAE-Sephadex. the size of the microspheres has been of the order or 40-60 µm in diameter.

The clearance of labelled microspheres from the nose has been studied in human volunteers using the standard technique of gamma scintigraphy (7). The microspheres were labelled with technetium-99m and applied to the nose in powder form using a nasal insufflator. Liquid and powder formulations were used as controls. The position of the noses of the volunteers was held constant on the collimator of the gamma camera using a specially designed template. Scintiscans were obtained over a suitable time period and regions of interest were created around the site of deposition in the nasal cavity. The time-activity profiles showed clearly that the nasal spray and powder formulations are cleared quite rapidly (with a time for 50% clearance (T_{50%} ) of 15 minutes). In contrast, the microsphere systems have a much longer clearance time. After 3 hours about 50% of the albumin and starch microspheres and 60% of the DEAE-Sephadex microspheres still remain at the site of application. The half-time of clearance from this initial deposition site for DEAE-Sephadex microspheres were calculated to be about 4 hours. At the present time we are exploring whether these microsphere systems will provide an enhancement of the bioavailability of selected drug substances to include peptides and proteins. We expect that a decreased clearance rate and the possible protection of labile drugs against enzymatic attack will significantly increase absorption efficiency.

In relation to controlled release systems and the nose it is interesting to note that Nagai and colleagues (8) have been able to increase the absorption of insulin after nasal application to dogs by using a gelling formulation. Insulin was mixed with a cellulosic material and Carbopol 934 (polyacrylic acid) and applied as a powder formulation. Similarly, Morimoto and colleagues (9) have used a nasal gel (once again polyacrylic acid) as a delivery system for insulin and calcitonin in rats. A significant decrease in plasma glucose levels obtained as compared to the normal formulation indicated an increase in the absorption efficiency.

A major problem in drug delivery is the effective absorption of high molecular weight materials such as proteins and peptides across biological membranes. Normally such molecules are not taken up by the body if administered to the gastrointestinal tract, to the buccal mucosa, to the rectal mucosa, the vaginal mucosa or given as an intranasal system.

As discussed above and by Chien and Chang (4) recent studies with insulin have demonstrated that the absorption of such a compound can be increased if it is given together with a so-called absorption enhancer. These absorption enhancing materials have included surfactants of the non-ionic type as well as various bile salt derivatives. An increased permeability of membranes in the presence of these types of surfactant materials is not unexpected, indeed the literature in the field of gastroenterology contains a wide range of such absorption promoters. (For a review see Davis *et al* (10). However, such materials may not be acceptable for the chronic administration of pharmacological agents because of their irritant effects on membranes. This includes not only the non-ionic variety of surface active agents but also bile salts and bile salt derivatives (e.g. fusidic acid).

It is an object of the present invention to provide a drug delivery system which enhances delivery of high molecular weight materials.

The present invention therefore provides a drug delivery system for transmucosal delivery including a plurality of microsphere particles containing an active drug and including a material associated with each particle which material has the property of increasing the bioavailability of the active drug across a mucosal membrane.

Preferably the particles are administered in the form of a powder by spraying and have bioadhesive properties.

The material which increases bioavailability should not produce any problems in terms of chronic toxicity because in vivo the material should be non-irritant and/or rapidly metabolised to a normal cell constituent that does not have any significant irritant effect. A preferred material is lysolecithin and other lysophosphatidyl compounds such as lysophosphatidyl-ethanolamine, lysophosphatidic acid etc. A suitable concentration is from 0.02 to 10% (w/v).

Embodiments of the present invention will now be described, by way of example, with reference to the accompanying drawings, in which:
Figure 1 illustrates in graphical form the effect of the use of the natural surfactant material on the uptake of a drug in a first experiment;
Figure 2 illustrates in graphical form the effect of the use of the natural surfactant material and the administration in the form of microspheres;
Figure 3 illustrates in graphical form the effect of the use of the use of the natural surfactant material in a rat study experiment;
Figures 4 and 5 respectively show plasma glucose levels for rabbits given intranasal doses of Zn and Na-insulin;
Figure 6 shows plasma glucose levels obtained for administration intranasally of insulin in different forms;
Figure 7 shows corresponding curves for plasma insulin levels;
Figure 8 shows data from rat experiments with hGH given intranasally; and
Figure 9 shows data from sheet experiments with hGH given intranasally.

Lysophosphatides are produced by the hydrolysis of phospholipids. Such materials are surface active and form micellar structures. In the present invention lysolecithin and other lysophosphatides are added to the active drug to act as a potential absorption enhancer for drug delivery. Lysophosphatidylcholine changes the permeability of membranes and allows the increased uptake of proteins and peptides including, for example, insulin, human growth hormone and other products of biotechnology and recombinant DNA methodologies. After administration the lysophosphatides are converted by the cells of the endothelial lining of the mucosa to the intact phosphatides which are normal cell components (see de Vries etal (11). (Lysolecithin itself is also present in cell membranes in very small quantities (12)). This rapid and efficient conversion of lysophosphatides into the complete phosphatide structure leads to much reduced adverse reactions and side effects in terms of irritation and toxicity.

The drug to be administered to a mucosal surface in the gastrointestinal tract, genital tract or the nose, eye or lung could be administered as a viscous solution, a suspension or a powder, together with lysolecithin or more preferably it should be administered in the form of a colloidal particle comprising a microsphere system. The advantage of using bioadhesive microsphere systems for administration to the nucosal surface is that such systems should allow a longer period of contact, especially if the microspheres are slowly degrading. This is particularly true for the nasal administration of drugs contained in microspheres produced from natural materials such as albumin, gelatin and especially starch. In some instances the longer contact time alone can provide a satisfactory improvement in biological availability.

A preferred material which increases bioavailability is the material lysophosphatidylcholine produced from egg or soy lecithin. Other lysophosphatidylcholines that have different acyl groups as well as lyso compounds produced from phosphatidylethanolamines and phosphatidic acid which have similar membrane modifying properties may be used. Acyl carnitines (e.g. Palmitoyl-DL Canitinechloride) is an alternative.

Other agents that would be appropriate for the present invention include chelating agents (EGTA, EDTA, alginates ) surface active agents ( especially nonionic materials), acyl glycerols, fatty acids and salts, tyloxapol and biological detergents listed in the SIGMA Catalog, 1988, page 316-321. Also agents that modify the membrane fluidity and permeability would be appropriate such as Enamines (e.g. phenylalanine enamine of ethyllacetoacecate), Malonates (e.g. diethyleneoxymethylene malonate) , Salicylates, Bile salts and analogues and fusidates. Suitable concentrations would be up to 10%.

The same concept of delivery of a drug incorporated into or onto a biadhesive microsphere with an added pharmaceutical adjuvant would apply to systems that contained active drug and mucolytic agent, peptidase inhibitors or irrelevant polypeptide substrate singly or in combination. A suitably mucolytic would be thiol containing compounds such as N-acetylcysteine and derivatives thereof. Peptide inhibitors include Actinonin, Amastatin, Antipain, Bestatin, Chloroacetyl-HOLeu-Ala-Gly-NH2, Diprotin A and B, Ebelactone A and B, E-64, Leupeptin, Pepstatin A, Phisphoramidon, H-Thr-(tBu)-Phe-Pro-Oh, Aprotinin, Kallikrein Inh.1, Chymostation, Benzamidine, Chymotrypsin Ing.11, trypsin Inh.111-0. Suitable concentrations would be from 0.01 to 5% (w/v).

The microspheres should be of a size between 10 and 100 µm and prepared from a biocompatible material that will gel in contact with the mucosal surface. Starch or starch derivative microspheres (cross linked if necessary) are a preferred material. Other microspheres include gelatin, albumin, dextran and its derivatives and collagen. Preparation of these microsphere systems is well described in the pharmaceutical literature (see for example Davis et al (13)). Emulsion and phase separation methods are both suitable. The final microspheres can be modified by chemical cross-linking or heat treatment. The active agent can be incorporated into the microspheres during their formulation or sorbed into/onto the system after preparation. The effectiveness of the system can be controlled by the physical nature of the microsphere matrix and e.g. the extent of the crosslinking. The microsphere delivery systems could also include microspheres made from the active peptide or protein itself such as insulin microspheres.

For example the preparation of the starch-insulin system was carried out by adding the freeze dried starch microspheres to a phosphate buffer solution (pH=7.3) containing the insulin and the enhancer system, mixing for 1 hour and freeze drying until a light powder was obtained. A typical concentration of insulin and enhancer system (e.g. lysolecithin) would be 1 IU/mg microsphere and 0.08mg/mg microspheres, respectively. The microspheres can be loaded with both less or more drug and enhancer system.

Using the combination of microspheres and enhancers, it has been found that the bioadhesive microsphere systems have the ability to greatly enhance the bioavailability of polar drugs when they are administered together with an enhancer system. This improvement is very much greater than the enhancement that can be achieved by the enhancer itself. This potentiation of enhancer action is believed to be due to the greater retention of the delivery system in the nasal cavity. The concept has been shown to be successful for different drugs such as gentamicin, insulin and growth hormone. The enhancer selected for these studies has been lysophosphatidylcholine (described above). The concept should work equally well with other enhancer system (see list elsewhere) and with other drugs such as;
Insulin (hexameric/dimeric/monomeric forms)
Glucagon
Growth Hormone (Somatotropin)
Polypeptides or their derivatives (preferably with a molecular weight from 1000 to 300,000)
Calcitonins and synthetic modifications thereof Enkephalins
Interferons (especially Alpha-2 Interferon for treatment of common colds)
LHRH and analogies (Nafarelin, Buserelin, Zolidex)
GHRH (Growth hormone releasing hormone)
Secretin
Nifedipin
Bradykin antagonists
GRF (Growth releasing factor)
THF
TRH (Thyrotropin releasing hormone)
ACTH analogues
IGF (Insulin like growth factors)
CGRP (Calcitonin gene related peptide)
Atrial Natriuretic Peptide
Vasopressin and analogues (DDAVP, Lypressin)
Antibiotics
Metaclopramide
Migraine treatment (Dihydroergotamine, Ergometrine, Ergotamine, Pizotizin)
Nasal Vaccones (Particularly AIDS vaccinces)
FACTOR VIII
Antibiotics and antimicrobial agents such as tetracycline hydrochloride, leucomycin, penicillin, penicillin derivatives and erythromycin, chemothrapeutic agents such as sulphathiazole and nitrofurazone; local anesthitics such as benzocaine; vasoconstrictors such as phenylephrine hydrochloride, tetrahydrozoline hydrochloride, naphazoline nitrate, oxymetazoline hydrochloride and tramazoline hydrochloride; cardiotonics such as digitalis and digoxin; vasodilators such as nitroglycerin and papaverine hydrochloride; antiseptics such as chlorhexidine hydrochloride, hexylresorcinol, dequalinium chloride and ethacridine; enzymes such as lysozyme chloride, dextranase; bone metabolism controlling agents such as vitamine D₃ and active vitamine D₃; sex hormones; hypotensives; sedatives; and anti-tumor agents.

Steroidal anti-inflammatory agents such as hydrocortisone, prednisone, fluticasone, predonisolone, triamcinolone, triamcinolone acetonide, dexamethasone, betamethasone, beclomethasone, and beclomethasone dipropionate; non-steroidal anti-inflammatory agents such as acetaminophen, aspirin, aminopyrine, phenylbutazone, mefenamic acid, ibuprofen, diclofenac sodium, indomethacin, colchicine, and probenocid; enzymatic antiinflammatory agents such as chymotrypsin and bromelain seratiopeptidase; anti-histaminic agents such as diphenhydramine hydrochloride, chloropheniramine maleate and clemastine; anti-allergic agents (antitussive-expectorant antasthmatic agents such as sodium cromoglycate, codeine phosphate, and isoprotereol hydrochloride.

### Administration

The microspheres can be administered via the nasal route using a nasal insufflator device. Example of these are already employed for commercial powder systems intended for nasal application (e.g. Fisons Lomudal System). Details of other devices can be found in the pharmaceutical literature (see for example Bell, A. Intranasal Delivery devices, in Drug Delivery Devices Fundamentals and Applications, Tyle P. (ed), Dekker, New York, 1988).

### Animal nasal delivery studies

The following studies of nasal delivery in animal models (rats, rabbits and sheep) has been carried out in order to substantiate the invention.

### Gentamicin:

The drug gentamicin was chosen as a model test substance. This polar compound is known to be poorly absorbed when administered into the nose (see for example Duchateau et al (17) and the biological availability can be enhanced by added bile salts.

### Rat Studies:

The in situ rat model of Hirai et al (14) was used as modified by Fisher et al (15). Male Wistar rats of about 200 g were anaesthetized by intraperitoneal injection of 60 mg/kg of Pentobarbitone (Sagatal, 60 mg/ml). The rats were tracheotomized, the oesophagus sealed and the carotid artery cannulated.

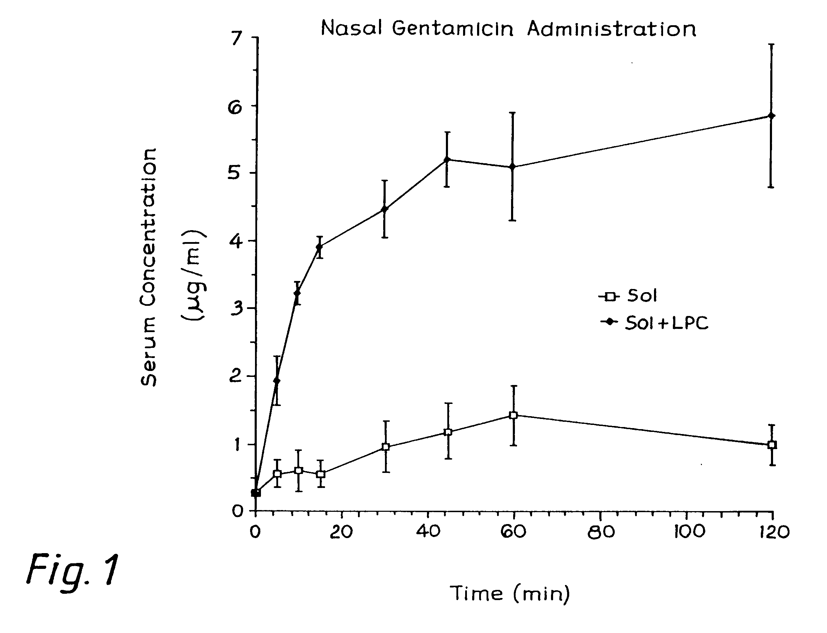

A volume of the gentamicin solution containing the drug with and without added lysophosphatidylcholine (LPC) (0.2% (w/v)) was instilled into the nasal cavity. Blood samples were withdrawn from the carotid artery at 0, 5, 10, 15, 30, 45, 60 and 120 min after drug administration. The gentamicin level was determined by the EMIT method (16). The effect of the LPC enhancer is demonstrated in Figure 1. The administration of gentamicin solution alone resulted in a poor bioavailability whereas the adding of the enhancer system gave rise to a five fold greater peak level. The AUC's (from t=0 to t=120 min) were 128 and 557 µg min/ml, respectively.

### Sheep studies:

Cross-bred (Suffolk and Texel) sheep were divided into groups of 3 and 2. The mean weight of the sheep was about 40 kg.

The animals were not fasted prior to the administration of gentamicin. An in-dwelling Viggo secalon universal central venous catheter of 1.2mm, i.d. with a secalon universal flow-switch was placed in the right jugular vein of each animal on the first day of the study and whenever necessary was kept patent by flushing with heparinised normal saline (50 IU/ml). The catheter was removed upon completion of the study. For intranasal administration, the sheep were sedated by an IV dose of Ketamine hydrochloride at 2 mg/kg to prevent sneezing during administration. The sedation lasted about 3 minutes. The animals that received gentamicin by the IV route were also sedated.

For the intranasal administration of solutions, a blueline umbilical cannula of 35 cm length (size 6 FG) was inserted into the nostril of the sheep to a preset depth of 10 cm before the delivery of the solution from a 1 ml syringe. For intranasal administration of powdered formulations, a BOC endotracheal tube (red rubber, cuffed) of 6.5 mm was loaded with the powder formulation and then inserted into the nostril of the sheep to a preset depth of 6 cm before blowing the powder into the nasal cavity.

The first group of sheep (n=2) was given 0.25 ml of gentamicin solution (386 mg/ml) (5.0 mg/kg) into each nostril. The second group (n=3) received 0.25 ml of gentamicin solution (386 mg/ml) (5.0 mg/kg) containing 2 mg/ml LPC into each nostril. The third group (n=3) received 5.0 mg/kg gentamicin and 0.2 mg/kg LPC in combination with starch microspheres (1.9 mg gentamicin/mg starch microspheres). The last group of sheep (n=3) was given 2 mg/kg gentamicin administered intravenously as a solution (40 mg/ml) through the jugular vein. Blood samples (2 ml) were collected through the jugular vein at 0, 8, 16, 24, 32, 45, 60, 90, 120, 180 and 240 min after drug administration. The serum was separated by centrifugation and the samples stored at -20°C awaiting analysis. No heparin was added to any of the samples. The gentamicin level was determined by the EMIT technique (16).

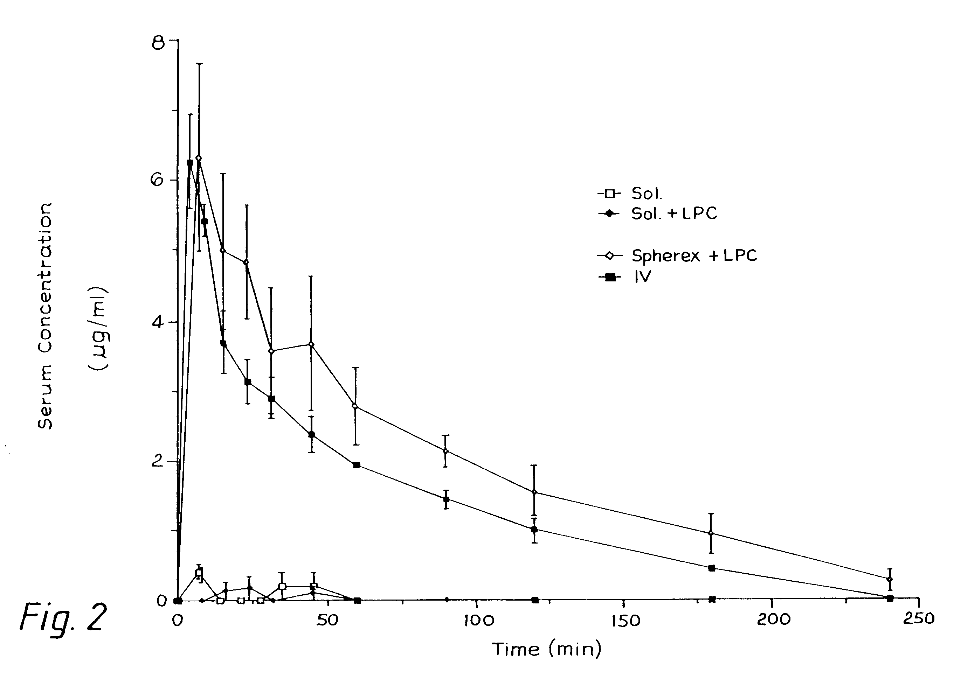

A dramatic effect is seen when the gentamicin plus enhancer are adminstered in the form of the starch microsphere formulation, the blood level peaking at 6.3 µg/ml as compared to 0.4 µg/ml for gentamicin solution. The combination of microspheres plus LPC enhancer provides a blood level-time profile that is very similar to that obtained when gentamicin is given intravenously (Figure 2).

Accounting for the doses administered the bioavailability for the intranasally adminstered gentamicin in combination with the LPC enhancer and gelling microsphere system is 57.3% as compared to the gentamicin given by IV dose.

### Insulin

In all animal studies the glucose plasma levels were analysed using the glucose oxidase method. The plasma insulin levels were determined for the rabbit and sheep experiments by means of radioimmune assay using a double-antibody technique.

### Rat Studies:

The Hirai's in situ model (as modified by Fisher) was used to study the nasal absorption of insulin using non-diabetic male Wistar Rats of 150 g fasted overnight. The rats were anaesthesized with an i.p. injection of 0.25 ml of Pentobarbitone (60 mg/ml).

A 250 IV/ml solution of Zinc (Zn)-human insulin was prepared in buffer (1/75 M Na₂HPO₄ ) of pH 7.3. In some experiments 0.2% (w/v) of LPC or for comparison 1% (w/v) Glycodeoxycholate (GDC) were added to the preparation as absorption enhancers. The experiments were performed in replicate (n=4). 10 µl was administered into the nasal cavity equivalent to 16.67 IV/kg (2.5 IV/rat). Blood samples (0.2 ml) were collected into 5 ml fluoride oxalate tubes at 10, 6 and 2 min pre-administration and at 5, 10, 20, 40, 60, 90, 120, 180, 240 and 300 min post-administration. The blood was replaced by saline administered through the jugular vein.

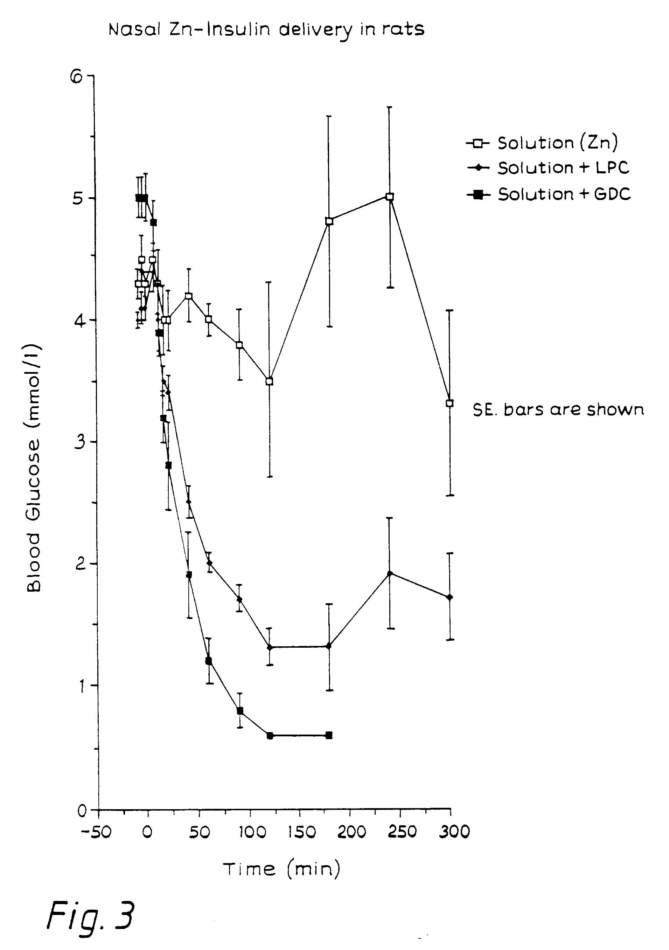

Figure 3 shows the glucose levels for rats given intranasal doses of Zn-insulin solution, Zn-insulin solution in combination with 0.2% (w/v) LPC or Zn-insulin solution in combination with 1% (w/v) GDC. The results indicate that insulin given intranasally as a simple solution is not effective in lowering the plasma glucose level whereas the addition of an enhancer system such as LPC causes a fast and significant drop in measured plasma levels. The LPC enhancer system in a concentration of 0.2% (w/v) can be seen to have a similar effect to 1% (w/v) bile salt in this in situ model where the cilia clearance mechanism is impaired.

### Rabbit studies:

Preparations of Zn-insulin (mainly hexamer form) or Na-insulin (mainly monomer/dimer forms) were administered nasally to rabbits either as free insulin or as a microsphere delivery system with lysophosphatidylcholine (LPC) as an enhancer. The experiments were performed in replicate (n=4).

Non-fasted New Zealand White female rabbits of average weight 3.5 kg were used in this study.

A 40 IU/ml Zn- or Na-human insulin solution was prepared in buffer (1/75 M Na₂HPO₄ ) of pH 7.3-7.4. In some experiments 0.2% (w/v) LPC was added.

A total of 200 µl of the solution (100 ul in each nostril) was administered intranasally equivalent to about 2.3 IU/kg using an Eppendorf pipette.

The rabbits were dosed s.c. with insulin at 0.8 IU/kg or 0.6 IU/kg from a 14 IU/ml or 10 IU/ml aqueous solution, respectively.

The dose of starch microspheres and insulin was fixed at 2.5 mg/kg and 2.5 IU/kg, respectively. The dose of LPC was 0.2 mg/kg. The average weight of the rabbits was 3.5 kg.

25 mg of microspheres were placed in a small glass vial and 250 µl of a 100 IU/ml insulin solution (Na- or Zn- insulin) was added followed by the 2 mg LPC and 250 µl of distilled water. The microspheres were then allowed to stand for 2h at room temperature in contact with the insulin solution before freeze drying.

Approximately 15 mg of the freeze dried powder from each individual vial was filled into the applicator tubing, and this was stored in a dessicator until use.

The rabbits were administered the suggested dose into the nasal cavity without sedation. Each rabbit was held on its back during, and for 10 second after, the application to ensure the delivery of the powdered formulation. Blood samples of 200 µl and 2 ml for glucose and insulin determination, respectively, were collected from the marginal ear vein at 10 and 5 min prior to the administration and at 5, 15, 30, 45, 60, 90, 120 and 180 min post-administration. For insulin analysis, the blood collected was mixed gently in 5 ml heparinised (Li Heparin) tubes. For glucose analysis, the blood collected was mixed gently in 5 ml fluoride oxalate tubes. The blood samples for glucose analysis were kept on crushed ice awaiting immediate analysis. The blood samples for insulin analysis were spun at 3000 rpm and the plasma collected was stored at -20°C awaiting analysis.

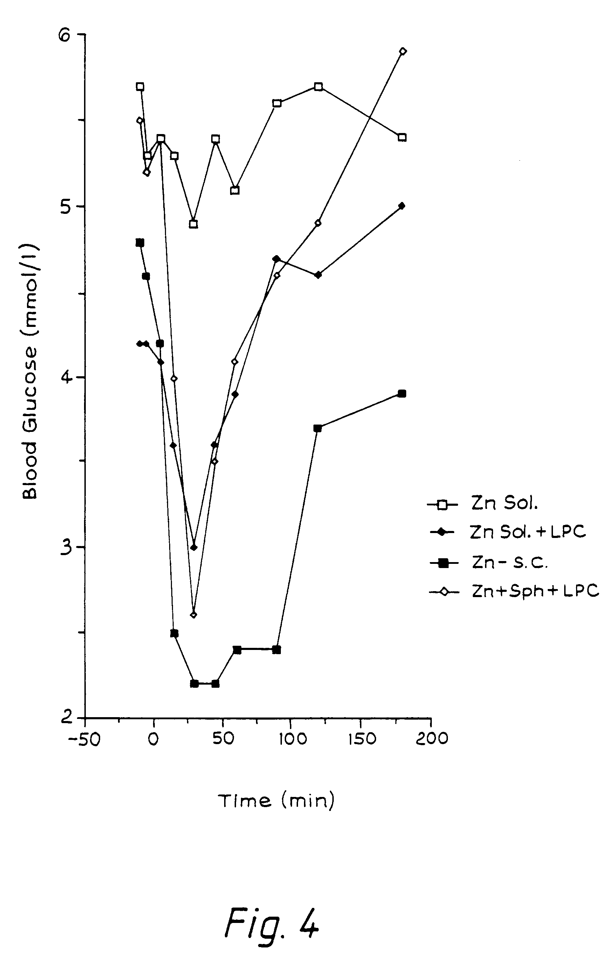

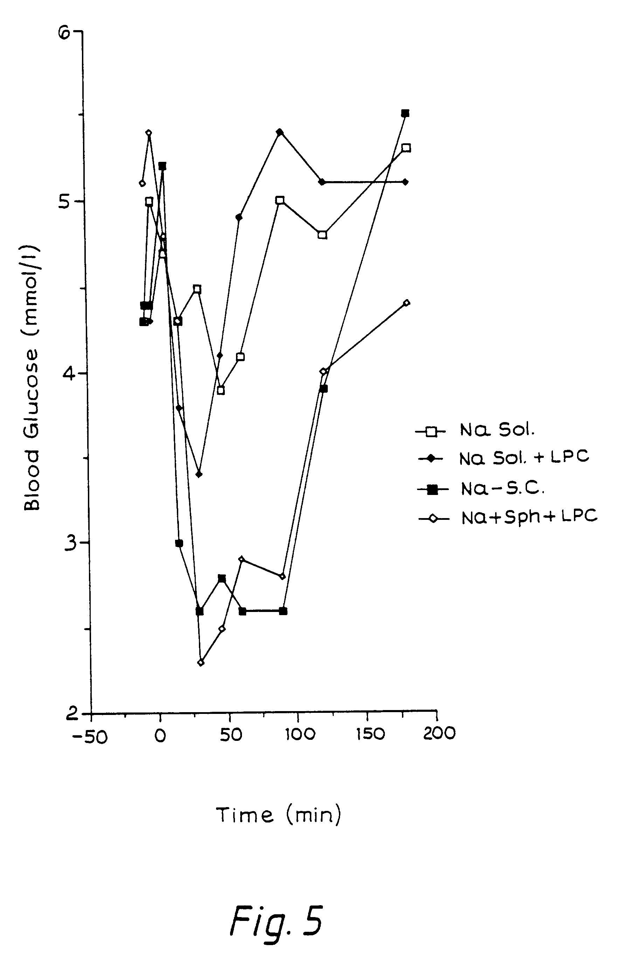

Figures 4 and 5 show the plasma glucose levels for rabbits given intranasal doses of Zn-insulin or Na-insulin, respectively as simple solutions, in simple solutions with 0.2% (w/v) LPC added or in combination with starch microspheres and LPC. Also shown is the plasma glucose levels for rabbits injected s.c. with Zn-insulin or Na-insulin. The results show that for both types of insulin (hexameric and monomeric/dimeric form) the administration of the insulin in combination with the LPC-enhancer system the plasma glucose levels are lowered significantly compared to the simple insulin solutions. However, even more drastic decreases in plasma glucose levels are seen when the insulin is administered in combination with the microspheres and enhancer system. The shape of the plasma glucose curves for the latter systems are suprisingly similar to the ones obtained for the s.c. administration, although the doses are 2.5 IU/kg compared to 0.6 IU/kg for the s.c. dosing.

### Sheep Studies

Zn-crystallised highly purified semisynthetic human insulin, each 1 mg of pure protein is equivalent to 28 IU insulin. Insulin solutions were prepared in 1/75M phosphate buffer (pH 7.3).

Fifteen cross-bred (Suffolk and Texel) sheep were used in this study. The animals were ear-tagged and weighed prior to the study: The mean weight in kg of the sheep (± S.D.) was 35.9
(±2.7). The animals were not fasted prior to insulin administration because it is difficult to achieve this in practice and because of the possibility of inducing insulin resistance in the animals. The latter term means that under such conditions the sheep blood glucose levels would not respond as readily to the insulin administered.

An in-dwelling Viggo secalon universal central venous catheter of 1.2 mm i.d. with a secalon universal flow-switch was placed in the right jugular vein of each animal on the first day of the study and whenever necessary, was kept patent by flushing it with heparinised normal saline (50IU/ml). This catheter was removed upon the completion of the study.

### Preparation of insulin solutions and powders:

Insulin stock solutions were prepared in 1/75 M phosphate buffer (pH 7.3). These were then used as liquid formulations for intravenous and intranasal administration, and also in the preparation of the lyophilised microsphere formulations. The latter were prepared by dispersing the required quantity of microspheres in the insulin solution (+ any LPC), stirring for 1 hour at room temperature, and then freeze-drying to obtain the powder formulation.

### Administration of insulin formulations:

Insulin was administered at 0.1 IU/kg via the intravenous route, at 0.2 IU/kg via the subcutaneous route, and at 2 IU/kg via the nasal route. Three sheep were used in each experiment:
(1) Intravenous administration of insulin as an aqueous solution prepared at 4IU/ml : 24/11/87.
(2) Intranasal administration of an aqueous solution, prepared at 200 IU/ml : 24/11/87.
(3) Intranasal administration of an aqueous solution, prepared at 200 IU/ml in combination with 0.2% LPC (0.02 mg/kg) : 24/11/87.
(4) Intranasal administration of insulin in combination with starch microspheres (2.5 mg/kg) and LPC (0.20 mg/kg) as a lyophilised powder. To prepare the formulation 500mg of Spherex were dispersed in 30 ml of 1/75M phosphate buffer (pH 7.3) containing 400 IU insulin and 40 mg LPC, mixed for 1h, and then freeze-dried : 26/11/87.
(5) Intranasal administration of starch microspheres (2.5 mg/kg) without insulin. To prepare the formulation, 500 mg of Spherex were dispersed in 30 ml of 1/75M phosphate buffer (pH 7.3), mixed for 1h, and then freeze-dried : 24/11/87.
(6) Subcutaneous administration of insulin as an aqueous solution prepared at 4.2IU/ml.

For intranasal administration of solutions, a blueline umbilical cannula of 35 cm length (size 6FG, Portex Ltd., Hythe, Kent, England) was inserted into the nostril of the sheep to a preset depth of 10 cm before the delivery of the solution from a 1 ml syringe. For intranasal administration of powdered formulations, a BOC endotracheal tube (red rubber, cuffed) of 6.5 mm was loaded with the powder formulation and then inserted into the nostril of the sheep to a preset depth of 6 cm before blowing the powder into the nasal cavity.

For intranasal administration, the sheep were sedated by an i.v. dose of Ketamine hydrochloride at 2 mg/kg. This was intended as a counter-measure against the animal sneezing during administration. The anaesthesia lasts for about 3 minutes. The animals which received insulin by the i.v. route were also sedated to counteract any possible effect of ketamine on the blood glucose or insulin levels measured.

Blood samples of 5 ml were collected onto crushed ice from the cannulated jugular vein of the sheep at 15 and 5 min prior to the insulin administration and at 5, 10, 15, 20, 30, 40, 50, 60, 75, 90, 120, 150, 180, and 240 min post-administration. Each blood sample was divided into two parts. For insulin analysis, the blood collected (2.5 ml) was mixed gently in 5 ml heparinised (Li Heparin) tubes. For glucose analysis, the blood collected (2.5 ml) was mixed gently in 5 ml fluoride oxalate tubes. All blood samples following withdrawal were maintained on crushed ice, awaiting centrifugation which was then performed at 4°C and 3000 rpm. The plasma collected was stored at -20°C awaiting insulin and glucose analysis (radioimmune assay for insulin).

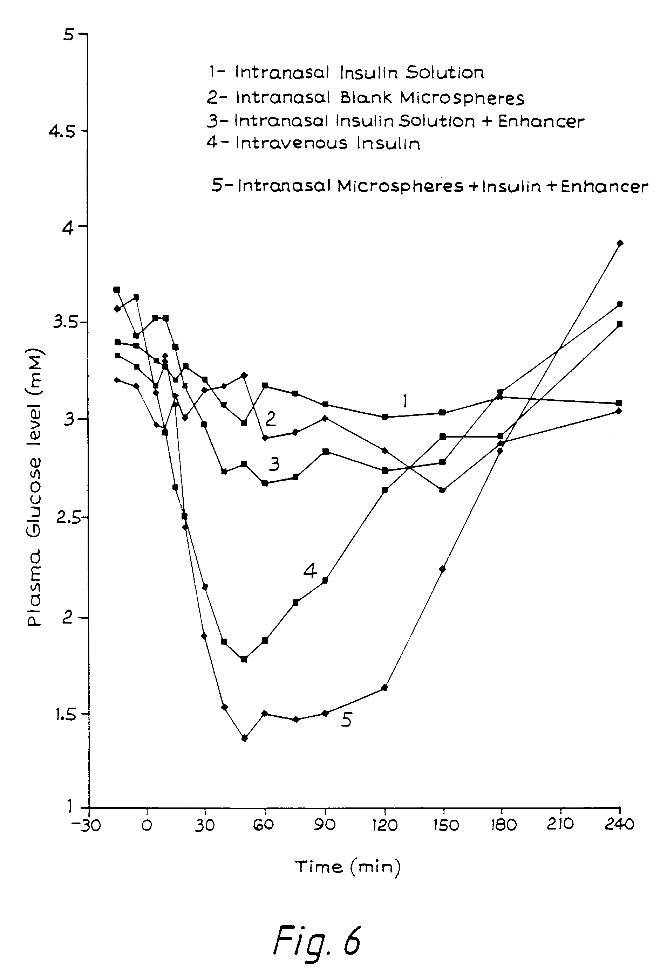

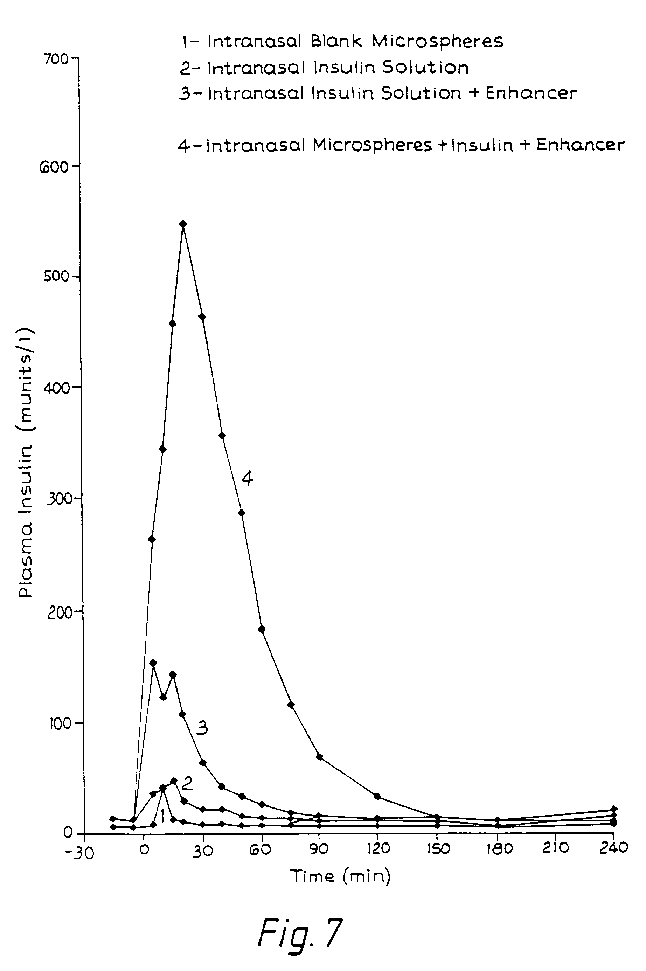

Figure 6 shows the plasma glucose levels obtained for the administration intranasally of a simple insulin solution, of blank starch microspheres, of insulin solution with added 0. 2% (w/v) LPC, insulin as a microsphere formulation in combination with LPC and the intravenous administration of insulin. Figure 7 shows the corresponding curves for plasma insulin levels. As seen in the rat and rabbit studies insulin administered intranasally as a simple solution does not have a significant effect on the plasma glucose level and the amount of insulin being absorbed via this route is indeed very low. Adding the enhancer system (LPC) to the formulation increases the amount of insulin appearing in the circulation and hence results in a somewhat lower plasma glucose level. The administration of the insulin in combination the starch microspheres and LPC results in a 693% increase in AUC of plasma insulin as compared to a simple nasal insulin solution. At the same time the peak insulin level is increased with 1040%. The sharp level peak appears at 15-20 min and decreases rapdily as for intravenous insulin. Considering the glucose levels obtained when administering the insulin-microsphere-enhancer system the shape of the plasma glucose profile is very similar to the one obtained for the intravenous insulin. The relative bioavailability for this sytem is about 25% as compared to a subcutaneous injection of insulin.

### Human growth hormone

For all experiments biosynthetic hGH was used. The plasma levels were analysed using a solid-phase 2-site sandwich-ELISA technique. Plasma was assayed in duplicate at a dilution of 1/10 against a standard solution of B-hGH (0.11-7.0ng/ml) prepared in antigen incubation buffer and also prepared in the appropriate dilution of plasma.

### Rat studies:

As before the experiments were performed using the rat in situ model described by Hirai and modified by Fisher.

Non-fasted male Wistar rats of about 200g were divided into groups of 4 and anaesthesized using an i.p. injection of 0.35 ml of pentobarbitone (60 mg/ml).

Three different hGH preparations were administered to the rats namely a 10 mg/ml solution of hGH in potassium phosphate buffer (1/75M), pH=7.2, as before with the addition of 0.05% (w/v) LPC and as before with the addition of 0.5% (w/v) LPC.

20 µl (1 mg/kg) of either of the three preparations were administered intranasally by means of a plastic tubing.

All experiments were performed in replicate. Blood samples, 20 drops were collected and kept on ice at times 0, 5, 10, 20, 30, 40, 50, 60, 90, 120, 180, 240 and 300 min after the application. The plasma was separated and stored at -20°C until analysis.

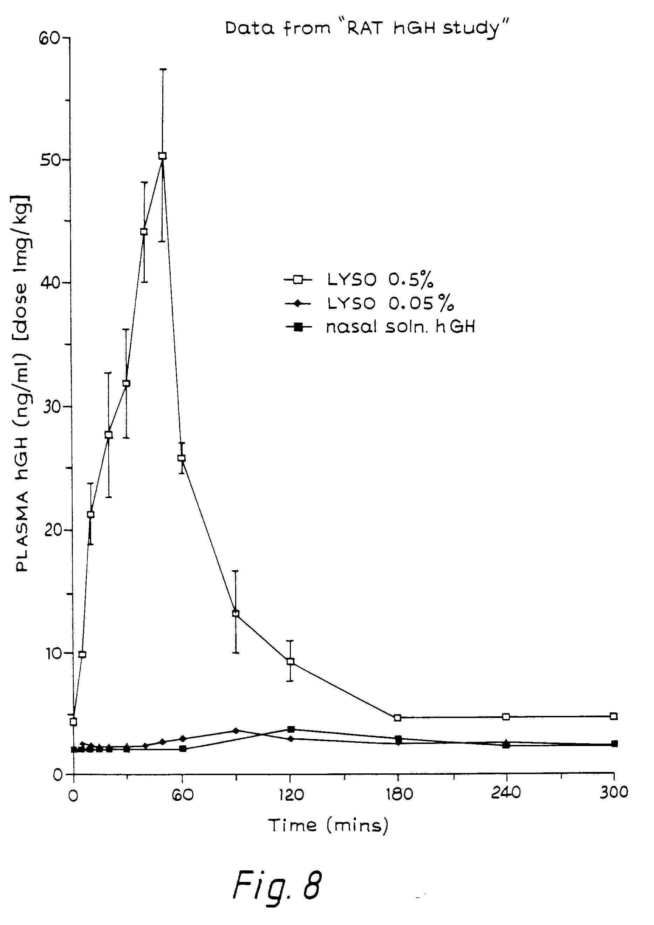

From Figure 8 it can be seen that hGH given intranasally as a solution without an enhancer system is not absorbed at any significant degree via the nasal membrane. However with the addition of 0.5% (w/v) LPC to the solution the resultant plasma level peaks are increased from about 3.5 ng/ml to about 57 ng/ml with a very significant increase in AUC. The addition of a very low concentration (0.05%) of LPC has apparently no effect on the absorption of hGH.

### Sheep studies:

Twelve cross-bred (Suffolk and Texel) sheep were used in this study. The animals were ear-tagged and weighed prior to the study:
The mean weight in kg of the sheep (± S.D.) was 35.8 (±3.0).

An in-dwelling Viggo secalon universal central venous catheter of 1.2 mm i.d. with a secalon universal flow-switch was placed in the right jugular vein of each animal on the first day of the study and whenever necessary, was kept patent by flushing it with heparinised normal saline (25IU/ml). This catheter was removed upon the completion of the study.

hGH was administered at 34.2ug/kg (0.1 IU/kg) via the subcutaneous route and at 307.5ug/kg (0.9 IU/kg) via the nasal route. Three sheep were used in each experiment:
(1) Subcutaneous administration of hGH as an aqueous solution prepared at 1.37mg/ml (4 IU/ml).
(2) Intranasal administration of hGH as an aqueous solution prepared at 17.57 mg/ml (51.43 IU/ml). A sheep of 40 kg would thus receive 0.35 ml of the formulation in each nostril (0.70 ml total).
(3) Intranasal administration of hGH in combination with starch microspheres (2.5 mg/kg) and LPC (0.20 mg/kg) as a lyophilised powder. To prepare the formulation, 500 mg of Spherex were dispersed in 30 ml of sterile distilled water containing 61.5 mg hGH (180 IU) and 40 mg LPC, mixed for 1h, and then freeze-dried :
For intranasal administration of solutions, a blueline umbilical cannular of 35 cm length was inserted into the nostril of the sheep to a preset depth of 10 cm before the delivery of the solution from a 1 ml syringe. For intranasal administration of powdered formulation, a BOC endotracheal tube (red rubber, cuffed) of 6.5 mm was loaded with the powder formulation and then inserted into the nostril of the sheep to a preset depth of 6 cm before blowing the powder into the nasal cavity.

For the intranasal studies, it is necessary to sedate the sheep by use of an i.v. dose of Ketamine hydrochloride at 2 mg/kg. This is intended as a counter-measure against the animal sneezing during administration. The anaesthesia lasts for about 3 minutes.

The animals which received hGH by the s/c route were also sedated to counter-act any possible effect of ketamine on the blood hGH levels measured.

Blood samples of 2 ml were collected in heparinised (Li Heparin) tubes onto crushed ice from the cannulated jugular vein of the sheep prior to the hGH administration and at 10, 20, 30, 40, 50, 60, 75, 90, 120, 150, 180, 240, and 300 min post-administration. The plasma collected by centrifugation (3000 rpm at 4°C) were stored at -20°C awaiting analysis by the ELISA technique.

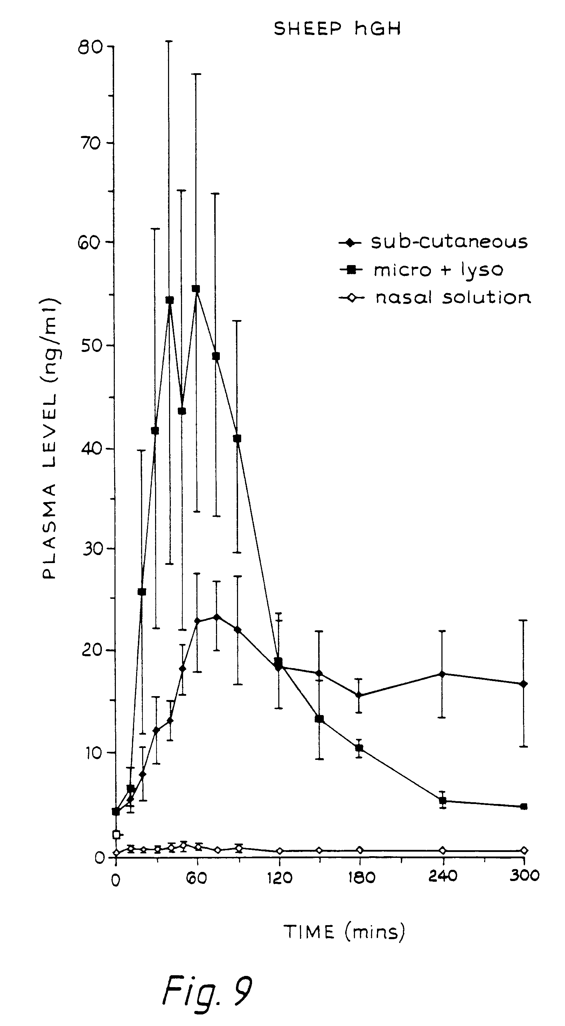

Figure 9 shows the obtained hGH levels for the intranasal administration of a simple hGH solution, the intranasal administration of hGH in combination with microspheres and LPC and the subcutaneous injection of hGH. It can be concluded from the results that the hGH administered intranasally as a simple solution is not absorbed by any significant extent. However, when the hGH is administered in combination with microspheres and the LPC enhancer system the hGH plasma level is increased considerably. Thus, the peak plasma level is increased from about 10 ng/ml to about 55 ng/ml. The bioavailability as compared to subcutaneous injection can be calculated to about 20%.

### References

1. Hussain, A., Hirai, S. and Bawarshi, R., Nasal absorption of natural contraceptive steroids in rats - progesterone absorption, J. Pharm. Sci. 70, 1981, 466-467.
2. Hussain, A., Hirai, S. and Bawarshi, R., Nasal absorption of propranolol from different dosage forms by rats and dogs, J. Pharm. Sci. 69, 1980. 1411-1413.
3. Salzman, R., Manson, J.E., Griffing, C.T., Kimmerle, R., Ruderman, N., McCall, A., Stoltz, E.I., Mullin, C., Small, D., Armstrong, J. and Melly, J.S., Intranasal aerosolized insulin, N. Eng. J. Med. 312, 1985, 1078-1084.
4. Chien, Y.W., and Chang, S.F., Intranasal Dry Delivery for Systemic Medications CRC Critical Reviews in Therapeutic Drug Carrier Systems 4, 67ug (1987).
5. Hanson, M., Gazdick, G., Cahill, J. and Augustine, M., Intranasal delivery of the peptide, salmon calcitonin, in S.S. Davis, L. Illum and E. Tomlinson: Advanced Delivery Systems for Peptides and Proteins, Plenum Press, London, 1986, pp. 233-242.
6. Gordon, G.F., Moses, A.G., Silver, R.D., Flier, G.F. and Carey, E., Nasal absorption of insulin: enhancement by hydrophobic bile salts, Proc. Natl. Acad. Sci. USA 82, 1985, 7419-7423.
7. Illum, L., Jorgenson, H., Bisgaard, H., Krogsgaard, O., and Rossing N., Bioadhesive microspheres as a potential nasal drug delivery system. Int. J. Pharmaceut. 39, 189-199 (1987).
8. Nagai, T., Nishimoto, Y., Nambu, N., Suzuki, Y. and Sekine, K., Powder dosage form of insulin for nasal administration, J. Control, Rel. 1, 1984, 15-22.
9. Morimoto, K., Morisaka, K. and Kamada, A., Enhancment of nasal absorption of insulin and calcitonin using polyacrylic acid gel, J. Pharm. Pharmacol. 37, 1985, 135-136.
10. S.S. Davis, L. Illum and E. Tomlinson (Eds). Delivery systems for peptide drugs, Plenum, New York, 1987.
11. de Vries, A.C.J., Batenburg, F.F. and van Golde, L.M.G. Lysophosphatidylcholine acyltransferase and lysophosphatidylcholine: lysophosphatidylcholine acyltransferase in alveolar type II cells from fetal rat lung. Biochem. Biophys. Acta 833 (1985) 93-99.
12. Christiansen, K. and Carlsen, J, Reconstitution of a protein into lipid vesicles using natural detergents. Biochim. Biophys. Acta 735 (1983) 225-233.
13. Davis S.S., Illum, L. McVie, J.G. and Tomlinson E. (eds) Microspheres and Drug Therapy, Pharmaceutical, Immunological and Medical Aspects, Elsevier Science Publishers B.V., Amsterdam, 1983.
14 Fisher, A.N., Brown, K., Davis, S.S., Par, G.D. and Smith D.A., The effect of molecular size on the nasal absorption of water soluble compounds by the albino rat, J. Pharm. Pharmacol. 39, 1987, 357-362.
15. Hirai, S., Yashiki, T., Matsuzawa, T. and Mima, H., Absorption of drugs from the nasal mucosa of rat, Int. J. Pharm. 7, 1981, 317-325.
16. O'Connell , M.B. , Hein, K. , Halstenson, C. and Matzke, G.R. Heparin interference with tobramycin, netilmicin and gentamicin concentrations determined by EMIT. Drug Intell. Glin. Pharm. 18 (1984) 503-504.
17. Duchateau, G.S.M.J.E., Zuidema, F. and Merkus, W.H.M. Bile salts and intranasal drug absorption. Int. J. Pharm. 31 (1986) 193-199.

## Claims

1. A drug delivery system for transmucosal delivery including a plurality of microsphere particles containing an active drug and including a material associated with each particle which material has the property of increasing the bioavailability of the active drug across a mucosal membrane.

2. A drug delivery system as claimed in Claim 1 in which the microspheres are of a size between 10 and 100 µm.

3. A drug delivery system as claimed in Claim 1 or 2 in which the material is a surfactant material.

4. A drug delivery system as claimed in Claim 1, 2 or 3 wherein the drug and material are incorporated during the process of microsphere preparation.

5. A drug delivery system as claimed in Claim 1, 2 or 3 wherein the drug and material are sorbed into or onto the microspheres after preparation thereof.

6. A drug delivery system as claimed in any one of the preceding claims wherein the microspheres are prepared from starch, starch derivatives, gelatin, albumin, collagen, dextran or dextran derivatives.

7. A drug delivery system according to Claim 6 wherein the microspheres are prepared from starch.

8. A drug delivery system as claimed in Claim 4 wherein the microspheres are formed from the active drug itself.

9. A drug delivery system as claimed in any one of the preceding claims wherein the microspheres are modified by a process of crosslinking.

10. A drug delivery system as claimed in any one of the preceding claims wherein the material which increases bioavailability is a lysophosphatidylcholine.

11. A drug delivery system as claimed in any one of Claims 3 to 9 wherein the surfactant is a non-ionic surfactant.

12. A drug delivery system as claimed in any one of the preceding claims for intranasal administration.

13. A drug delivery system as claimed in any one of the preceding claims wherein the drug is a biologically active polypeptide or derivative thereof with a molecular weight from 1000 to 300,000.

14. A drug delivery system as claimed in Claim 13 wherein the polypeptide is insulin or growth hormone.

## Patentansprüche

1. Arzneimittelabgabesystem zur schleimhautdurchgängigen Abgabe mit einer Vielzahl von Mikrokügelchenteilchen, enthaltend ein aktives Arzneimittel und ein mit jedem Teilchen verbundenes Material mit der Fähigkeit zur Steigerung der Bioverfügbarkeit des aktiven Arzneimittels durch eine Schleimhautmembran hindurch.

2. Arzneimittelabgabesystem nach Anspruch 1, wobei die Mikrokügelchen eine Größe zwischen 10 und 100 µm aufweisen.

3. Arzneimittelabgabesystem nach Anspruch 1 oder 2, wobei das Material aus einem oberflächenaktiven Material besteht.

4. Arzneimittelabgabesystem nach Anspruch 1, 2 oder 3, wobei das Arzneimittel und das Material während der Mikrokügelchenherstellung eingearbeitet wurden.

5. Arzneimittelabgabesystem nach Anspruch 1, 2 oder 3, wobei das Arzneimittel und das Material in die oder auf die Mikrokügelchen nach ihrer Herstellung sorbiert wurden.

6. Arzneimittelabgabesystem nach einem der vorhergehenden Ansprüche, wobei die Mikrokügelchen aus Stärke, Stärkederivaten, Gelatine, Albumin, Kollagen, Dextran und Dextranderivaten hergestellt sind.

7. Arzneimittelabgabesystem nach Anspruch 6, wobei die Mikrokügelchen aus Stärke hergestellt sind.

8. Arzneimittelabgabesystem nach Anspruch 4, wobei die Mikrokügelchen aus dem aktiven Arzneimittel selbst hergestellt sind.

9. Arzneimittelabgabesystem nach einem der vorhergehenden Ansprüche, wobei die Mikrokügelchen durch einen Vernetzungsprozeß modifiziert wurden.

10. Arzneimittelabgabesystem nach einem der vorhergehenden Ansprüche, wobei das die Bioverfügbarkeit steigernde Material aus einem Lysophosphatidylcholin besteht.

11. Arzneimittelabgabesystem nach einem der Ansprüche 3 bis 9, wobei das oberflächenaktive Mittel aus einem nichtionischen oberflächenaktiven Mittel besteht.

12. Arzneimittelabgabesystem nach einem der vorhergehenden Ansprüche zur intranasalen Verabreichung.

13. Arzneimittelabgabesystem nach einem der vorhergehenden Ansprüche, wobei das Arzneimittel aus einem biologisch aktiven Polypeptid oder Derivat desselben mit einem Molekulargewicht von 1000 bis 300 000 besteht.

14. Arzneimittelabgabesystem nach Anspruch 13, wobei das Polypeptid aus Insulin oder einem Wachstumshormon besteht.

## Revendications

1. Système d'administration d'un médicament pour une administration à travers les muqueuses incluant une pluralité de particules en forme de microsphères contenant un médicament actif et incluant une matière associée à chaque particule cette matière ayant la propriété d'augmenter la bioexploitabilité du médicament actif à travers une membrane de muqueuse.

2. Système d'administration d'un médicament selon la revendication 1 dans lequel les microsphères sont d'une dimension comprise entre 10 et 100 µm.

3. Système d'administration d'un médicament selon la revendication 1 ou 2 dans lequel la matière est une matière tensio-active.

4. Système d'administration d'un médicament selon la revendication 1, 2 ou 3 dans lequel le médicament et la matière sont incorporés durant le processus de préparation des microsphères.

5. Système d'administration d'un médicament selon la revendication 1, 2 ou 3 dans lequel le médicament et la matière sont absorbés dans ou sur les microsphères après leur préparation.

6. Système d'administration d'un médicament selon l'une quelconque des revendications précédentes dans lequel les microsphères sont préparées à partir d'amidon, de dérivés d'amidon, de gélatine, albumine, collagène, dextrane ou dérivés de dextrane.

7. Système d'administration d'un médicament selon la revendication 6 dans lequel les microsphères sont préparées à partir d'amidon.

8. Système d'administration d'un médicament selon la revendication 4 dans lequel les microsphères sont formées à partir du médicament lui-même actif.

9. Système d'administration d'un médicament selon l'une quelconque des revendications précédentes dans lequel les microsphères sont modifiées par un processus de réticulation.

10. Système d'administration d'un médicament selon l'une quelconque des revendications précédentes dans lequel la matière qui accroit la bioexploitabilité est un lisophosphatidylcholine.

11. Système d'administration d'un médicament selon l'une quelconque des revendications 3 à 9 dans lequel l'agent tensio-actif est un agent tensio-actif non ionique.

12. Système d'administration d'un médicament selon l'une quelconque des revendications précédentes en vue d'une administration intranasale.

13. Système d'administration d'un médicament selon l'une quelconque des revendications précédentes dans lequel le médicament est un polypeptide biologiquement actif ou un dérivé de celui-ci ayant un poids moléculaire allant de 1000 à 300 000.

14. Système d'administration d'un médicament selon la revendication 13 dans lequel le polypeptide est l'insuline ou une hormone de croissance.
